(19) Europäisches Patentamt
European Patent Office
Office européen des brevets

(11) **EP 3 329 227 B1**

(12) **EUROPÄISCHE PATENTSCHRIFT**

(45) Veröffentlichungstag und Bekanntmachung des Hinweises auf die Patenterteilung:
**15.04.2020   Patentblatt 2020/16**

(21) Anmeldenummer: **16750121.2**

(22) Anmeldetag: **26.07.2016**

(51) Int Cl.:
*G01F 23/284* (2006.01)   *G01F 23/296* (2006.01)
*G01N 9/00* (2006.01)   *G01N 11/16* (2006.01)
*G01N 33/03* (2006.01)   *G01N 33/28* (2006.01)

(86) Internationale Anmeldenummer:
**PCT/EP2016/067712**

(87) Internationale Veröffentlichungsnummer:
**WO 2017/017076 (02.02.2017 Gazette 2017/05)**

(54) **VORRICHTUNG ZUR BESTIMMUNG UND/ODER ÜBERWACHUNG ZUMINDEST EINER PROZESSGRÖSSE**

DEVICE FOR DETERMINING AND/OR MONITORING AT LEAST ONE PROCESS VARIABLE

DISPOSITIF POUR DÉTERMINER ET/OU SURVEILLER AU MOINS UNE GRANDEUR DE PROCESSUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2015   DE 102015112543**

(43) Veröffentlichungstag der Anmeldung:
**06.06.2018   Patentblatt 2018/23**

(73) Patentinhaber: **Endress+Hauser SE+Co. KG**
**79689 Maulburg (DE)**

(72) Erfinder:
• **BLÖDT, Thomas**
**79585 Steinen (CH)**

• **BRENGARTNER, Tobias**
**79312 Emmendingen (DE)**
• **MACK, Benjamin**
**79539 Lörrach (DE)**
• **KLÖFER, Peter**
**79585 Steinen (DE)**

(74) Vertreter: **Andres, Angelika Maria**
**Endress+Hauser (Deutschland) AG+Co. KG**
**PatServe**
**Colmarer Strasse 6**
**79576 Weil am Rhein (DE)**

(56) Entgegenhaltungen:
WO-A1-2008/006391   WO-A1-2008/006391
DE-A1- 10 057 974   DE-A1-102004 059 050
DE-A1-102005 050 400   US-A1- 2006 031 030

**Beschreibung**

Die Erfindung betrifft eine Vorrichtung zur Bestimmung und/oder Überwachung zumindest einer Prozessgröße eines Mediums.

Entsprechende Feldgeräte in der Prozess- und/oder Automatisierungstechnik basieren je nach zu bestimmender Prozessgröße auf vielen unterschiedlichen Prinzipien, welche an sich aus dem Stand der Technik bekannt sind. Dabei kann ein einzelnes Feldgerät entweder dazu in der Lage sein, eine einzelne Prozessgröße, oder aber auch, insbesondere simultan, mehrere Prozessgrößen, zu erfassen. Für eine umfassende Charakterisierung der, insbesondere physikalischen und/oder chemischen, Eigenschaften eines Mediums müssen jedoch in der Regel mehrere Feldgeräte parallel eingesetzt werden.

Ein Feldgerät umfasst typischerweise zumindest ein direkt oder indirekt zumindest teilweise und zumindest zeitweise mit dem Prozess in Berührung kommendes Sensorelement sowie eine Elektronikeinheit, welche beispielsweise der Signalerfassung, -auswertung und/oder -speisung dient. Beispielsweise umfasst das Sensorelement eines vibronischen Füllstandsmessgeräts typischerweise zumindest eine mechanisch schwingfähige Einheit, wie beispielsweise eine Schwinggabel, einen Einstab oder eine Membran. Diese wird im Betrieb mittels einer Antriebs-/Empfangseinheit, häufig in Form einer elektromechanischen Wandlereinheit, zu mechanischen Schwingungen angeregt, welche wiederum beispielsweise ein piezoelektrischer Antrieb oder ein elektromagnetischer Antrieb sein kann. Es sei an dieser Stelle darauf verwiesen, dass es sich bei der Antriebs-/Empfangseinheit je nach Ausgestaltung des Messgeräts entweder um eine separate Antriebseinheit und eine separate Empfangseinheit, oder um eine kombinierte Antriebs-/Empfangseinheit handeln kann.

Vibronische Füllstandsmessgeräte werden von der Anmelderin in großer Vielfalt hergestellt und beispielsweise unter der Bezeichnung LIQUIPHANT oder SOLIPHANT vertrieben. Die zugrundeliegenden Messprinzipien sind im Prinzip aus einer Vielzahl von Veröffentlichungen bekannt. Die Antriebs-/Empfangseinheit regt die mechanisch schwingfähige Einheit mittels eines elektrischen Anregesignals zu mechanischen Schwingungen an. Umgekehrt kann die Antriebs-/Empfangseinheit die mechanischen Schwingungen der mechanisch schwingfähigen Einheit empfangen und in ein elektrisches Empfangssignal umwandeln. Dabei ist die Antriebs-/Empfangseinheit in vielen Fällen Teil eines rückgekoppelten elektrischen Schwingkreises, mittels welchem die Anregung der mechanisch schwingfähigen Einheit zu mechanischen Schwingungen erfolgt. Beispielsweise muss für eine resonante Schwingung die Schwingkreisbedingung, gemäß welcher die Summe aller Verstärkungen im Schwingkreis, bzw. der Verstärkungsfaktor $\geq 1$ ist und alle im Schwingkreis auftretenden Phasen ein Vielfaches von 360° ergeben, erfüllt sein.

Zur Anregung und Erfüllung der Schwingkreisbedingung muss eine bestimmte Phasenverschiebung zwischen dem Anregesignal und dem Empfangssignal gewährleistet sein. Deshalb wird häufig ein vorgebbarer Wert für die Phasenverschiebung, also ein Sollwert für die Phasenverschiebung zwischen dem Anregesignal und dem Empfangssignal eingestellt. Hierfür sind aus dem Stand der Technik unterschiedlichste Lösungen, sowohl analoge als auch digitale Verfahren, bekannt geworden. Prinzipiell kann die Einstellung der Phasenverschiebung beispielsweise durch Verwendung eines geeigneten Filters vorgenommen werden, oder auch mittels eines Regelkreises auf eine vorgebbare Phasenverschiebung, den Sollwert, geregelt werden. Aus der DE102006034105A1 ist beispielsweise bekannt geworden, einen einstellbaren Phasenschieber zu verwenden. Die zusätzliche Integration eines Verstärkers mit einstellbarem Verstärkungsfaktor zur zusätzlichen Regelung der Schwingungsamplitude wurde dagegen in der DE102007013557A1 beschrieben. Die DE102005015547A1 schlägt die Verwendung eines Allpass-Filters vor. Die Einstellung der Phasenverschiebung ist außerdem mittels eines sogenannten Frequenzsuchlaufs möglich, wie beispielsweise in der der DE102009026685A1, DE102009028022A1, und DE102010030982A1 offenbart. Die Phasenverschiebung kann aber auch mittels einer Phasenregelschleife (engl. Phase-Locked-Loop, PLL) auf einen vorgebbaren Wert geregelt werden. Ein hierauf basierendes Anregungsverfahren ist Gegenstand der DE00102010030982A1.

Sowohl das Anregesignal als auch das Empfangssignal sind charakterisiert durch ihre Frequenz f, Amplitude A und/oder Phase $\Phi$. Entsprechend werden Änderungen in diesen Größen üblicherweise zur Bestimmung der jeweiligen Prozessgröße, wie einem vorgegebenen Füllstand, eines Durchflusses, der Dichte und/oder der Viskosität, herangezogen. Im Falle eines vibronischen Grenzstandschalters für Flüssigkeiten wird beispielsweise unterschieden, ob die schwingfähige Einheit von der Flüssigkeit bedeckt ist oder frei schwingt. Diese beiden Zustände, der Freizustand und der Bedecktzustand, werden dabei beispielsweise anhand unterschiedlicher Resonanzfrequenzen, also einer Frequenzverschiebung, bei Vorliegen einer vorgebbaren Phasenverschiebung zwischen Anregesignal und Empfangssignal unterschieden. Die Dichte und/oder Viskosität wiederum lassen sich mit einem derartigen Messgerät nur ermitteln, wenn die schwingfähige Einheit vom Medium bedeckt ist.

Zur Bestimmung und/oder Überwachung der Dichte eines Mediums sind aus der DE10057974A1 ein Verfahren sowie eine Vorrichtung bekannt geworden, mittels welcher/welchem der Einfluss von zumindest einer Störgröße, beispielswese der Viskosität, auf die Schwingungsfrequenz der mechanisch schwingfähigen Einheit zu ermittelt und entsprechend kompensiert wird. In der DE102006033819A1 ist ferner beschrieben, eine vorgebbare Phasenverschiebung zwischen dem Anregesignal und dem Empfangssignal einzustellen, bei welcher Auswirkungen von Änderungen der Viskosität des Mediums auf die mechanischen Schwingungen der mechanisch schwingfähigen Einheit vernachlässigbar sind. Dabei wird die Dichte im Wesentlichen nach der Formel

$$\rho_{Med} = \frac{1}{K}\left[\left(\frac{f_{0,Vak} + C \cdot t + A \cdot t^2}{f_{T,P,Med}}\right)^2 \cdot (1 + D \cdot p) - 1\right]$$

bestimmt, wobei K die Dichteempfindlichkeit der mechanisch schwingfähigen Einheit ist, $f_{0,vak}$ die Frequenz der mechanischen Schwingungen im Vakuum, C und A den linearen, bzw. quadratischen Temperaturkoeffizienten der mechanisch schwingfähigen Einheit, t die Prozesstemperatur, $f_{T,P,Med}$ die Frequenz der mechanischen Schwingungen im Medium, D den Druckkoeffizienten, und p der Druck des Mediums.

Die Viskosität eines Mediums mittels eines vibronischen Sensors kann wiederum anhand der Frequenz-Phase-Kurve ($\Phi$=g(f)) bestimmt werden, wie beispielweise in der DE10050299A1 beschrieben. Dieses Vorgehen basiert auf der Abhängigkeit der Dämpfung der schwingfähigen Einheit von der Viskosität des jeweiligen Mediums. Dabei gilt, dass je geringer die Viskosität ist, desto steiler fällt die Frequenz-Phase-Kurve ab. Um den Einfluss der Dichte auf die Messung zu eliminieren, wird die Viskosität anhand einer durch zwei unterschiedliche Werte für die Phase verursachten Frequenzänderung bestimmt, also mittels einer Relativmessung. Dazu können entweder zwei unterschiedliche Phasenwerte eingestellt und die zugehörige Frequenzänderung bestimmt werden, oder es wird ein vorgegebenes Frequenzband durchfahren und festgestellt, wenn zumindest zwei vorgegebene Phasenwerte erreicht werden. Aus der DE102007043811A1 ist darüber hinaus bekannt geworden, aus einer Änderung der Eigenfrequenz und/oder Resonanzfrequenz und/oder der Phasenlage auf eine Änderung der Viskosität zu schließen und/oder aufgrund entsprechend hinterlegter Abhängigkeiten der Schwingungen der schwingfähigen Einheit von der Viskosität des jeweiligen Mediums die Viskosität zu bestimmen. Auch bei diesem Vorgehen muss die Abhängigkeit der Bestimmung der Viskosität von der Dichte des Mediums berücksichtigt werden.

Zusammenfassend ermöglicht der Einsatz eines vibronischen Füllstandsmessgeräts sowohl die Bestimmung und/oder Überwachung eines vorgebbaren Füllstands, insb. eines Grenzstands, als auch die Bestimmung der Dichte und/oder Viskosität des Mediums, falls die schwingfähige Einheit von Medium bedeckt ist. Weitere zur Charakterisierung eines Mediums benötigte Prozessgrößen sind nur unter Verwendung zumindest eines weiteren und auf einem anderen Prinzip basierenden Messgeräts ermittelbar.

Aus der WO2008/006391A1 ist beispielsweise ein Füllstandsmessumformer nach dem Radarprinzip mit einer auf dem vibronischen Messprinzip basierenden Diagnoseeinrichtung zur Erkennung einer Verschmutzung der Antenne des Füllstandsmessumformers bekannt geworden. Aus der DE102004059050A1 ist wiederum eine Vorrichtung bekannt geworden, bei welcher eine Sondeneinheit mittels des vibronischen Messprinzips zu Schwingungen angeregt wird, um zusätzlich zur eigentlichen Prozessgröße eine Aussage über Ansatz bzw. Korrosion an der Sondeneinheit treffen zu können.

Die DE102005050400A1 beschreibt ein Messgerät zur Bestimmung und/oder Überwachung der Masse eines Mediums in einem Behälter oder des Massedurchflusses eines Mediums durch ein Rohr, bei welchem unterschiedliche Messvorrichtungen, wie beispielsweise ein Füllstands- und Dichtemessgerät oder ein Volumendurchflussmessgerät und ein Dichtemessgerät, kombiniert werden.

Auch aus der US2006/0031030A1 und der DE10057974A1 sind wiederum Messgeräte und Verfahren zur Bestimmung unterschiedlicher Eigenschaften von Medien bekannt geworden, bei welchem ein nach dem vibronischen Messprinzip arbeitender Sensor bekannt eingesetzt wird.

Der vorliegenden Erfindung liegt deshalb die Aufgabe zugrunde, eine Vorrichtung zur Bestimmung und/oder Überwachung zumindest einer Prozessgröße mit einem erweiterten Anwendungsbereich bereitzustellen.

Diese Aufgabe wird gelöst durch eine Vorrichtung zur Bestimmung und/oder Überwachung zumindest einer Prozessgröße eines Mediums in einem Behälter mit zumindest

- einer mechanisch schwingfähigen Einheit, und
- einer Antriebs-/Empfangseinheit zur Anregung der mechanisch schwingfähigen Einheit zu mechanischen Schwingungen mittels eines elektrischen Anregesignals und zum Empfangen und Umwandeln der mechanischen Schwingungen in ein elektrisches Empfangssignal
- einer Regeleinheit, welche dazu ausgestaltet ist, das Anregesignal ausgehend vom Empfangssignal zu erzeugen, und eine vorgebbare Phasenverschiebung zwischen dem Anregesignal und dem Empfangssignal einzustellen,
- einer elektromagnetisch schwingfähigen Einheit
- einem aktiven Element zur Erzeugung und/oder Aufrechterhaltung der elektromagnetischen Schwingungen in der elektromagnetisch schwingfähigen Einheit, welches aktive Element zusammen mit der elektromagnetisch schwingfähigen Einheit einen Oszillator bildet

- einer Auskoppeleinheit, welche dazu ausgestaltet ist, über das aktive Element ein Ausgangssignal abzugreifen, und
- einer Auswerteeinheit,

welche Auswerteeinheit dazu ausgestaltet ist, die zumindest eine Prozessgröße aus dem Empfangssignal und/oder aus dem Ausgangssignal zu bestimmen,
wobei zumindest eine mechanisch schwingfähige Einheit (4) in Form einer Schwinggabel mit einem ersten Gabelzinken (4a) und einem zweiten Gabelzinken (4b) und zumindest eine elektromagnetisch schwingfähige Einheit (7) mit zumindest einer ersten Leiterbahn (7a) und einer zweiten Leiterbahn (7a) vorgesehen sind, und
wobei die erste Leiterbahn (7a) zumindest einer der zumindest einen elektromagnetisch schwingfähigen Einheit (7) zumindest teilweise im Innenraum des ersten Gabelzinkens (4a) und die zweite Leiterbahn (7b) dieser elektromagnetisch schwingfähigen Einheit (7) zumindest teilweise im Innenraum des zweiten Gabelzinkens (4a) der zumindest einen mechanischen schwingfähigen Einheit (4) angeordnet ist oder
wobei die erste (7a) und zweite Leiterbahn (7b) zumindest einer der zumindest einen elektromagnetisch schwingfähigen Einheit (7) zumindest teilweise im Innenraum desselben Gabelzinkens (4a,4b) einer der zumindest einen mechanischen schwingfähigen Einheit (4) angeordnet sind oder
wobei die erste und/oder zweite Leiterbahn zumindest einer elektromagnetisch schwingfähigen Einheit innerhalb eines Gehäuses der Antriebs-/Empfangseinheit angeordnet sind.

Erfindungsgemäß ist die mechanisch schwingfähige Einheit zumindest Teil eines ersten Sensorelements und die elektromagnetisch schwingfähige Einheit zumindest Teil eines zweiten Sensorelements. Bei der erfindungsgemäßen Vorrichtung handelt es sich um eine Kombination aus einem vibronischen Füllstandsmessgerät und einem Messgerät in Form eines sogenannten Mikrowellen- Resonators, bzw. eines -Schwingsystems oder -Oszillators. Letzterer wird im Folgenden auch als Mikrowellen-Sensor bezeichnet. Vorteilhaft lässt sich auf diese Weise ein gegenüber einem herkömmlichen vibronischen Füllstandsmessgerät deutlich erweiterter Anwendungsbereich erzielen. Dies betrifft sowohl die Zugänglichkeit einer vergrößerten Anzahl an bestimmbaren Prozessgrößen sowie daraus mittels mathematischer Zusammenhänge ableitbarer

Messgeräte in Form eines Mikrowellen-Sensors sind aus dem Stand der Technik an sich bekannt und beispielsweise in den Offenlegungsschriften DE102012104075A1, DE102013112025A1 und DE102013112026A1 beschrieben. Bei einem entsprechenden Messgerät ist die jeweilige Prozessgröße anhand eines elektromagnetischen Feldes, welches durch die zumindest teilweise und/oder zeitweise Anwesenheit eines Mediums beeinflusst wird, ermittelbar.

Dazu ist ein sogenanntes frequenzbestimmendes Element, das ist in diesem Fall die elektromagnetisch schwingfähige Einheit, eines Oszillators, insbesondere eines Hochfrequenz-Oszillators mit Frequenzen im Bereich von 10MHz bis etwa 10GHz, derart angeordnet, dass es sich zumindest zeitweise und/oder teilweise in direkter Umgebung zum jeweiligen Medium bzw. Messmedium befindet. In Abhängigkeit des jeweiligen Mediums verändern sich dann die Schwingungseigenschaften des Oszillators, anhand derer die Bestimmung der jeweiligen Prozessgröße erfolgen kann.

Der Oszillator weist zumindest zur Erzeugung und Aufrechterhaltung der elektromagnetischen Schwingungen ein sogenanntes aktives Element auf, welches beispielsweise einem Verstärker entspricht. Das aktive Element ist insbesondere derart ausgelegt, dass zum einen eine elektromagnetische Schwingung innerhalb des Oszillators aufrechterhalten werden kann und zum anderen ein der Schwingungen im Oszillator entsprechendes Ausgangssignal über ein sogenanntes Auskoppelelement, im vorliegenden Falle Teil der Auskoppeleinheit, ausgekoppelt werden kann, ohne das Schwingungsverhalten des Oszillators zu beeinflussen. Anhand des Ausgangssignals wird schließlich die jeweilige Prozessgröße ermittelt. Bei dem Hochfrequenz-Oszillator kann es sich unter anderem um einen rückgekoppelten Oszillator oder auch um einen Reflexoszillator handeln. Der Aufbau eines geeigneten Hochfrequenz-Oszillators kann beispielsweise der Offenlegungsschrift DE102011078060A1 entnommen werden.

Im Folgenden werden beispielhaft einige bevorzugte Ausgestaltungen für ein erfindungsgemäßes Messgerät mit den sich jeweils ergebenden spezifischen Vorteilen beschrieben. Diese Ausführungen bilden dabei keineswegs eine abschließende Liste für mögliche Anwendungen und Ausgestaltungen.

In einer Ausgestaltung ist die mechanisch schwingfähige Einheit eine Membran, ein Einstab, oder eine Schwinggabel. Die mechanisch schwingfähige Einheit kann vorteilhaft aus einem Metall, aus einer Keramik oder aus einem Kunststoff gefertigt sein. Je nach Positionierung der elektromagnetisch schwingfähigen Einheit kann sich insbesondere die Verwendung eines Die mechanisch schwingfähige Einheit kann vorteilhaft aus einem Metall, aus einer Keramik oder aus einem Kunststoff gefertigt sein. Je nach Positionierung der elektromagnetisch schwingfähigen Einheit kann sich insbesondere die Verwendung eines Kunststoffes für die mechanisch schwingfähige Einheit als besonders vorteilhaft erweisen. Wird die elektromagnetisch schwingfähige Einheit beispielsweise zumindest teilweise zumindest innerhalb eines Teilbereichs der mechanisch schwingfähigen Einheit positioniert, so bietet ein Kunststoff zum einen eine hohe Transmission für Mikrowellenstrahlung. Zum anderen stellt der die elektromagnetisch schwingfähige Einheit zumindest teilweise umgebende Kunststoff eine wirksame Barriere für das jeweilige Medium, und somit beispielsweise einen Schutz vor Korrosion der elektromagnetisch schwingfähigen Einheit.

In einer Ausgestaltung umfasst die Antriebs-/Empfangseinheit zumindest ein piezoelektrisches Element oder zumindest eine Spule. Für piezoelektrische Antriebs-/Empfangseinheiten vibronischer Sensoren sind unterschiedlichste Ausgestaltungen bekannt geworden. Eine entsprechende elektromechanische Wandlereinheit mit zumindest einem piezoelektrischen Element umfasst zumindest eine Sendeeinheit oder Sendeelektrode und eine Empfangseinheit oder Empfangselektrode. In einigen Fällen ist darüber hinaus noch zumindest eine, beispielsweise als Massepotential dienende oder auch floatende, Referenzelektrode vorgesehen. Beispielhafte Ausgestaltungen sind im Falle eines sogenannten Bimorph-Antriebs unter anderem in der EP0751380B1 offenbart. Ein sogenannter Stapel-Antrieb ist dagegen beispielsweise in den Schriften EP0875741 B1, EP1134038A1, EP1277243B1 und EP19969005B1 beschrieben und ein sogenannter Vier-Quadranten-Antrieb ist unter anderem Gegenstand der EP1281051B1.

Dagegen erfolgt im Falle einer sogenannten elektromagnetische Antriebs-/Empfangseinheit die Umwandlung elektrischer Energie in mechanische Energie über ein magnetisches Wechselfeld, mittels welchem eine periodische Kraft auf die mechanisch schwingfähige Einheit übertragen wird. Auch für dieses Prinzip einer elektromechanischen Wandlereinheit, welche in der Regel zumindest eine Spule und einen Magneten umfasst, sind zahlreiche Ausgestaltungen bekannt, wie beispielsweise die in den Schriften WO 2007/113011 und WO 2007/114950 A1 beschriebenen.

In einer weiteren Ausgestaltung ist die elektromagnetisch schwingfähige Einheit als Wellenleiter, insb. als Koaxialkabel, Hohlraumleiter, oder Mikrostreifenleitung, als Patchantenne, als Gradientenleiter, insbesondere dielektrischer Gradientenleiter oder als Zweidrahtleitung ausgebildet. Grundsätzlich ist die elektromagnetisch schwingfähige Einheit derart beschaffen, dass sich elektromagnetische Wellen in ihr ausbreiten können. Im Falle einer Ausgestaltung als Wellenleiter kann die elektromagnetisch schwingfähige Einheit beispielsweise in einer Kabelform vorliegen, wie Streifenleitungen, wie beispielsweise eine Mikrostreifenleitung bekannt. Als Patchantenne wird in der Regel eine Antennenform bezeichnet, bei welcher eine häufig rechteckförmige Metallfläche als Resonator wirkt. Weiterhin sei unter einer Zweidrahtleitung beispielsweise eine sogenannte Bandleitung verstanden, welche gelegentlich auch als Stegleitung bezeichnet wird. Ein Gradientenleiter ist schließlich beispielsweise ein Lichtwellenleiter, insbesondere eine Multimode-Glasfaser. Bevorzugt handelt es um eine Multimode-Glasfaser mit einem vorgebbaren Gradientenindex-Profil entspricht. Für eine solche Multimode-Glasfaser werden sogenannte Gradientenindexfasern, oder auch Gradientenfasern, verwendet, für welche der Brechungsindex vom Faserkern nach außen hin kontinuierlich abfällt. Ein Gradientenleiter kann vorteilhaft vollständig aus einem Kunststoff aufgebaut werden.

In einer bevorzugten Ausgestaltung ist die zumindest eine Prozessgröße gegeben durch einen Füllstand oder Grenzstand des Mediums in dem Behälter, durch die Viskosität, die Dichte, die Permittivität, die Permeabilität, den dielektrischen Verlustfaktor, den Eiweißgehalt und/oder Fettgehalt eines Mediums, insb. eines Lebensmittels, oder durch den Wasseranteil in einem Öl. Diese Liste der ermittelbaren Prozessgrößen ist dabei keinesfalls abschließend. Mit der erfindungsgemäßen Vorrichtung kann anhand beider schwingfähiger Einheiten ein vorgebbarer Füllstand ermittelt werden. Darüber hinaus bietet die elektromagnetisch schwingfähige Einheit im Falle vergleichsweise kleiner Behälter auch die Möglichkeit, einen kontinuierlichen Füllstand zu erfassen. Die Dichte und/oder Viskosität des Mediums lassen sich anhand einer Auswertung der mechanischen Schwingungen der mechanisch schwingfähigen Einheit bestimmen. Dagegen lassen sich die Permittivität, auch als dielektrische Leitfähigkeit bezeichnet, bzw. die Permeabilität, oder auch magnetische Leitfähigkeit, welche jeweils ein Maß für die Durchlässigkeit eines bestimmten Mediums für elektrische bzw. magnetische Felder bezeichnet, mittels der elektromagnetisch schwingfähigen Einheit ermitteln, beispielsweise mittels des komplexen Brechungsindex, welcher ebenfalls bestimmbar ist. Der ebenfalls anhand der elektromagnetisch schwingfähigen Einheit ermittelbare dielektrische Verlustfaktor wiederum ist ein Maß für die Dissipation elektromagnetischer Energie innerhalb des Oszillators, und damit ein Maß für den Grad der Dämpfung der sich ausbreitenden elektromagnetischen Wellen.

Der Eiweißgehalt und/oder Fettgehalt eines Mediums kann wiederum aus einer Kombination der Prozessgrößen der Primittivität, dem dielektrischen Verlustfaktor, dem Transmissionsgrad und Reflexionsgrad sowie der Dichte und/oder Viskosität bestimmt werden. Dabei tritt in Bezug auf die elektromagnetisch schwingfähige Einheit im Falle eines Fettes eine vergleichsweise geringe Permittivität bei vergleichsweise geringem dielektrischen Verlustfaktor auf, während im Falle eines Eiweißes eine vergleichsweise geringe Permittivität bei vergleichsweise hohem dielektrischen Verlustfaktor auftritt. Die Bestimmung des Wasseranteils in einem Öl wiederum ist ausführlich in der DE102013112025A1 dargestellt. Bezüglich des Salzgehalts eines Mediums gilt, dass (Koch-)salz eine Permittivität, bzw. eine Dielektrizitätszahl von 5,9 unabhängig von der Temperatur aufweist. Weiterhin tritt in Bezug auf die elektromagnetisch schwingfähige Einheit ein vergleichsweise hoher dielektrischer Verlustfaktor bei vergleichsweise geringer Transmission auf. Werden weiterhin noch die Dichte des Mediums und/oder die Temperatur des Mediums berücksichtigt, lässt sich aus der Abweichung der für ein bestimmtes Medium ermittelten Dielektrizitätszahl vom Wert 5,9 der Salzgehalt eines Mediums, beispielsweise eines Lebensmittels, ermitteln.

Für die konkrete geometrische Ausgestaltung einer erfindungsgemäßen Vorrichtung sind viele verschiedene Möglichkeiten denkbar, von denen in der Folge ein paar besonders bevorzugte Varianten beschrieben werden. Die Vielfalt möglicher Ausgestaltungen betrifft dabei sowohl die Anordnung der zumindest zwei Sensorelemente, also der zumindest einen mechanisch schwingfähigen Einheit und der zumindest einen elektromagnetisch schwingfähigen Einheit als auch die Ausgestaltung zumindest einer Elektronikeinheit, welche unter anderem die der mechanisch schwingfähigen Einheit zugeordnete Regeleinheit als auch die der elektromagnetisch schwingfähigen Einheit zugeordnete Auskoppeleinheit sowie das aktive Element umfasst. Ferner kann der zumindest einen Elektronikeinheit noch die zumindest eine Auswerteeinheit zugeordnet werden, mittels welcher die zumindest eine Prozessgröße, beispielsweise anhand eines auf einem Mikroprozessor hinterlegten und ablaufenden Auswertealgorithmus, bestimmt werden.

In einer besonders bevorzugten Ausgestaltung ist die elektromagnetisch schwingfähige Einheit an zumindest einem Teilbereich oder innerhalb zumindest eines Teilbereichs der mechanisch schwingfähigen Einheit, oder gemeinsam mit, innerhalb, oder als Teil der Antriebs-/Empfangseinheit angeordnet. Es handelt sich hierbei um eine platzsparende Ausgestaltung, bei welcher gewährleistet ist, dass die elektromagnetisch schwingfähige Einheit in unmittelbarer Umgebung zum jeweiligen Medium angeordnet ist. Die verwendeten Materialien müssen in diesem Falle an die jeweilige geometrische Anordnung derart angepasst sein, dass sich innerhalb der elektromagnetisch schwingfähigen Einheit ausbreitenden elektromagnetischen Wellen zumindest teilweise in das Medium ausbreiten können. Die mechanisch schwingfähige Einheit und/oder zumindest ein Teil der Antriebs-/Empfangseinheit sollten also insbesondere eine hohe Transmission für die elektromagnetischen Wellen aufweisen. Insbesondere für eine derartige Ausführung der vorliegenden Erfindung ist gegebenenfalls die Verwendung eines Kunststoffes zumindest für einen Teil der mechanisch schwingfähigen Einheit und/oder zumindest eines Gehäuses der Antriebs-/Empfangseinheit von Vorteil.

Erfindungsgemäß ist eine mechanisch schwingfähige Einheit in Form einer Schwinggabel mit einem ersten und einem zweiten Gabelzinken und zumindest eine elektromagnetisch schwingfähige Einheit mit zumindest einer ersten Leiterbahn und einer zweiten Leiterbahn vorgesehen.

In dieser Hinsicht ist es einerseits möglich, dass die erste Leiterbahn zumindest einer der zumindest einen elektromagnetisch schwingfähigen Einheit zumindest teilweise im Innenraum des ersten Gabelzinkens und die zweite Leiterbahn dieser elektromagnetisch schwingfähigen Einheit zumindest teilweise im Innenraum des zweiten Gabelzinkens einer der zumindest einen mechanischen schwingfähigen Einheit angeordnet ist. Alternativ können die erste und zweite Leiterbahn zumindest einer der zumindest einen elektromagnetisch schwingfähigen Einheit zumindest teilweise im Innenraum desselben Gabelzinkens der zumindest einen mechanischen schwingfähigen Einheit angeordnet sein. Ebenfalls ist es von Vorteil, wenn die erste und/oder zweite Leiterbahn zumindest einer elektromagnetisch schwingfähigen Einheit innerhalb eines Gehäuses der Antriebs-/Empfangseinheit angeordnet sind.

Es versteht sich von selbst, dass neben den genannten Varianten noch weitere Ausgestaltungen möglich sind, welche ebenfalls unter die vorliegende Erfindung fallen. Beispielsweise können die zumindest erste und zweite Leiterbahn der elektromagnetisch schwingfähigen Einheit unterschiedliche Geometrien, insbesondere in Bezug auf ihre Breite und/oder Länge, aufweisen. Die Leiterbahnen sind bevorzugt aus Kupfer gefertigt, jedoch können selbstverständlich auch andere Materialien verwendet werden. Weiterhin ist es möglich, mehrere elektromagnetisch schwingfähige Einheiten innerhalb einer mechanisch schwingfähigen Einheit und/oder innerhalb der Antriebs-/Empfangseinheit anzuordnen. Durch eine unterschiedliche Positionierung und Ausrichtung relativ zueinander lässt sich das Anwendungsspektrum in Bezug auf die ermittelbaren Prozessgrößen nochmals erweitern, vergleiche auch die in der DE102013112025A1 beschriebenen Varianten.

Eine besonders bevorzugte Ausgestaltung beinhaltet, dass zumindest zwei elektromagnetisch schwingfähige Einheiten vorgesehen sind, wobei die zumindest zwei elektromagnetisch schwingfähigen Einheit derart angeordnet sind und die Auswerteeinheit dazu ausgestaltet ist, aus einem Vergleich zumindest eines ersten Ausgangssignals der ersten elektromagnetisch schwingfähigen Einheit und einem zweiten Ausgangssignal der zweiten elektromagnetisch schwingfähigen Einheit auf einen Ansatz an der zumindest einen mechanisch schwingfähigen Einheit zu schließen. Beispielsweise ist die mechanisch schwingfähige Einheit eine Schwinggabel Im Falle von Ansatzbildung zwischen den beiden Gabelzinken beginnen sich die mittels der zwei elektromagnetisch schwingfähigen Einheiten ermittelten Ausgangssignale zu unterscheiden. Dann kann beispielsweise ein Grenzwert für die Abweichung der beiden Ausgangssignale voneinander festgelegt und bei Überschreiten dieses Grenzwerts eine Meldung und/oder Warnung generiert werden, dass sich Ansatz gebildet hat. Die Detektion von Ansatz zwischen den Gabelzinken einer mechanisch schwingfähigen Einheit in Form einer Schwinggabel ist mittels eines herkömmlichen vibronischen Messgeräts nicht ohne Weiteres möglich. Die Überlegungen zur Ansatzdetektion lassen sich ferner leicht auf andere Ausgestaltungen der mechanisch schwingfähigen Einheit übertragen.

In einer weiteren Ausgestaltung ist zumindest eine elektromagnetisch schwingfähige Einheit in einem Gehäuse der Antriebs-/Empfangseinheit angeordnet, wobei die Auswerteeinheit dazu ausgestaltet ist, aus dem Ausgangssignal der elektromagnetisch schwingfähigen Einheit auf das Eindringen von Medium in das Gehäuse der Antriebs-/Empfangseinheit zu schließen. Auch in diesem Falle würde das Eindringen von Medium eine Änderung im Ausgangssignal der jeweiligen elektromagnetisch schwingfähigen Einheit hervorrufen. Beispielsweise kann das Eindringen von Medium in das Gehäuse der Antriebs-/Empfangseinheit anhand einer deutlichen, insbesondere sprunghaften, Erhöhung der Dielektrizitätszahl erkannt werden. Wieder kann dann beispielsweise im Falle des Überschreitens eines vorgebbaren Grenzwerts für die Änderung des Ausgangssignals eine Meldung und/oder Warnung generiert werden.

Weiterhin ist es von Vorteil, wenn zusätzlich ein Temperatursensor zur Erfassung der Temperatur des Mediums vorhanden ist. Dabei kann es sich beispielsweise um ein Widerstandselement, insbesondere um ein Platinelement, insb. z. B. ein sogenanntes PT100 oder PT1000 -Element, einen Heißleiter, insbesondere einen sogenannten NTC-Widerstand bzw. einen NTC-Thermistor (NTC =Negative Temperature Coefficient), um einen Kaltleiter, insbesondere einen sogenannten PTC-Widerstand bzw. einen PTC-Thermistor (PTC =Positive Temperature Coefficient) oder um einen sogenannten IC-Temperaturfühler bzw. IC-Temperatursensor handeln. Die Möglichkeit, die Temperatur als weitere Prozessgröße zu erfassen, ergibt viele messtechnische Vorteile, zum einen in Bezug auf die Menge an ermittelbaren Prozessgrößen, aber auch in Bezug auf die Messgenauigkeit. Sowohl einzelne Prozessgrößen, wie beispielsweise im Falle wässriger Medien deren Dielektrizitätszahl, als auch prozessnah angeordnete elektronische Komponenten der Vorrichtung, insbesondere in Bezug auf deren Performance, können eine Temperaturabhängigkeit aufweisen.

Neben den vielfältigen Ausgestaltungsmöglichkeiten für die zumindest zwei Sensorelemente sind auch in Bezug auf die elektronischen Komponenten der erfindungsgemäßen Vorrichtung viele verschiedene Varianten denkbar.

Eine herkömmliche Elektronikeinheit für einen vibronischen Sensor umfasst üblicherweise zumindest eine Regeleinheit und eine Auswerteeinheit. Die Antriebs-/Empfangseinheit ist in der Regel in unmittelbarer Umgebung zur jeweiligen mechanisch schwingfähigen Einheit angeordnet. Gleichzeitig ist sie jedoch in vielen Fällen Teil eines rückgekoppelten elektrischen Schwingkreises, mittels welchem die Anregung der mechanisch schwingfähigen Einheit zu mechanischen Schwingungen erfolgt. Mittels der Auswerteeinheit wird das jeweils von der mechanisch schwingfähigen Einheit empfangene Empfangssignal bezüglich seiner Frequenz, Phase und/oder Amplitude ausgewertet, und anhand zumindest einer dieser Größen die jeweilige Prozessgröße bestimmt.

Dagegen umfasst eine herkömmliche Elektronikeinheit für einen Mikrowellen-Sensor zumindest ein aktives Element, eine Auskoppeleinheit und eine Auswerteeinheit. Das aktive Element ist entweder direkt, oder über eine geeignete Verbindung, insbesondere über eine Steckverbindung, beispielsweise zur Verbindung zweier gleich- oder verschiedenartig ausgestalteter Wellenleiter, mit der elektromagnetisch schwingfähigen Einheit verbunden. Das aktive Element muss jedoch nicht in unmittelbarer Umgebung zum jeweiligen Medium angeordnet sein. Das aktive Element kann somit beispielsweise in einem separaten, die übrigen, insbesondere elektronischen, Komponenten des Messgeräts enthaltenden Gehäuse untergebracht sein. Das aktive Element ist ferner mit der Auskoppeleinheit verbunden, welche das jeweils ausgekoppelte Ausgangssignal einer Auswerteeinheit zuführt, welche das Ausgangssignal zumindest in Bezug auf dessen Frequenz auswertet und anhand der Frequenzauswertung die jeweilige Prozessgröße bestimmt. Der Mikrowellen-Sensor kann in zwei Betriebsmodi betrieben werden, in einem kontinuierlichen oder in einem gepulsten Modus, wobei der gepulste Modus deutlich energiesparender ist. Varianten für die Ausgestaltung einer geeigneten Auswerteeinheit sind beispielsweise in den Offenlegungsschriften DE102012104075A1 oder DE102013112025A1 beschrieben.

Es ist nun einerseits möglich, die elektronischen Komponenten einer erfindungsgemäßen Vorrichtung nach der jeweiligen schwingfähigen Einheit, also nach Sensorelement, separiert anzuordnen und zu kontaktieren. Es können jedoch auch mehrere Komponenten zusammengefügt bzw. in einer gemeinsamen Elektronikeinheit angeordnet werden. Auch die elektrischen Kontaktierungen der zumindest zwei Sensorelemente können zumindest teilweise zusammengelegt werden.

Eine mögliche Ausgestaltung sieht vor, dass zumindest zwei Elektronikeinheiten und eine Auswerteeinheit vorgesehen sind, wobei die erste Elektronikeinheit zumindest die Regeleinheit umfasst, wobei die zweite Elektronikeinheit zumindest das aktive Element und die Auskoppeleinheit umfasst, und wobei die Auswerteeinheit dazu ausgestaltet ist, aus dem Empfangssignal und/oder dem Ausgangssignal zumindest eine Prozessgröße zu bestimmen. Jede der beiden Elektronikeinheiten ist in diesem Falle mit der übergeordneten Auswerteeinheit elektrisch verbunden.

Alternativ sind gemäß einer anderen möglichen Ausgestaltung zumindest zwei Elektronikeinheiten vorgesehen, wobei die erste Elektronikeinheit zumindest die Regeleinheit und eine erste Auswerteeinheit, welche zumindest aus dem Empfangssignal zumindest eine Prozessgröße ermitteln kann, umfasst, und wobei die zweite Elektronikeinheit zumindest das aktive Element, die Auskoppeleinheit und eine zweite Auswerteeinheit, welche zumindest aus dem Ausgangssignal zumindest eine Prozessgröße ermitteln kann umfasst. Die zumindest zwei Auswerteeinheiten können jedoch auch separat von den restlichen elektronischen Komponenten der jeweils zumindest zwei Elektronikeinheiten angeordnet sein. Die zumindest zwei Elektronikeinheiten können ferner dazu ausgestaltet sein, miteinander zu kommunizieren. Insbesondere kann zumindest zur Datenübertragung zu einem übergeordneten Prozessleitsystem und/oder die Beaufschlagung der Sensorelemente, im Falle, dass die mittels beider Auswerteeinheiten bestimmten Prozessgrößen über dieselben Leitungen übertragen werden, nach dem Master/Slave Prinzip erfolgen.

Um eine gegenseitige Beeinflussung des Messetriebes in Bezug auf die mechanisch schwingfähige Einheit und die elektromagnetisch schwingfähige Einheit zu verhindern schwingt in einer bevorzugten Ausgestaltung die elektromagnetisch schwingfähige Einheit mit einer Frequenz im Bereich von 100MHz bis 100GHz die mechanisch schwingfähige Einheit mit einer Frequenz im Bereich von 100Hz bis 100kHz.

Im Falle, dass die Kontaktierung der beiden Sensorelemente mit den jeweiligen elektronischen Komponenten zumindest teilweise über dieselbe Leitung oder dieselben Leitungen erfolgt, ist es von Vorteil, wenn eine Triggereinheit vorgesehen ist, welche Triggereinheit dazu ausgestaltet ist, zumindest die Auskopplung des Ausgangssignals auf ein bestimmtes anhand des Anregesignals als Funktion der Zeit ermittelbares Zeitintervall, insbesondere ein einen Nulldurchgang oder einen Extrem-Wert des Anregesignal umfassendes Zeitintervall, zu triggern. In diesem Falle wird die elektromagnetisch schwingfähige Einheit in einem gepulsten Modus betrieben.

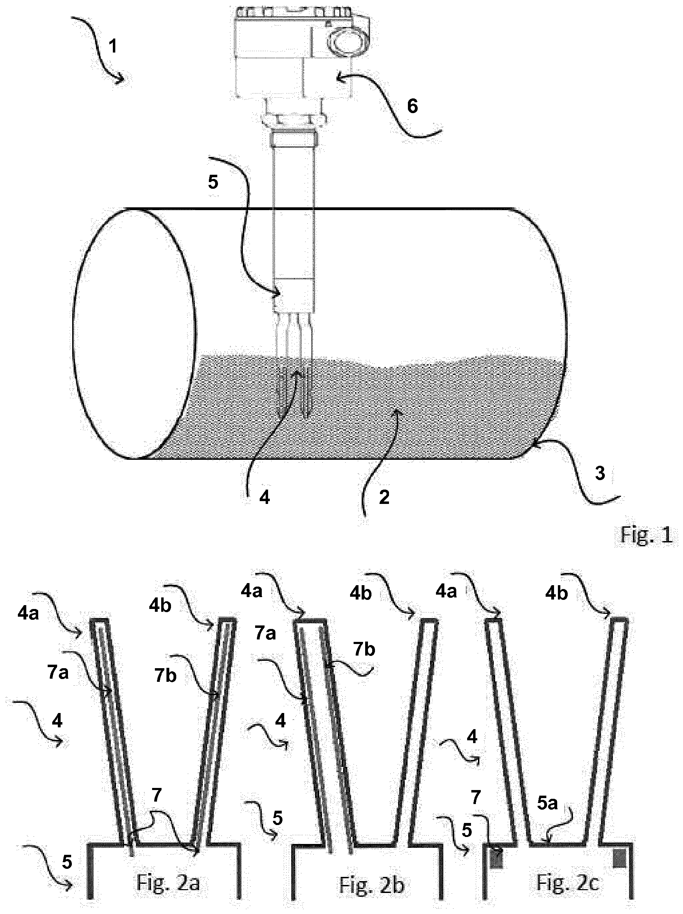

Die Erfindung sowie ihre vorteilhaften Ausgestaltungen werden nachfolgend anhand der Figuren Fig. 1 - Fig. 5 näher beschrieben. Es zeigt:

Fig. 1: eine schematische Skizze eines vibronischen Sensors gemäß Stand der Technik,

Fig. 2: drei schematische Zeichnungen einer erfindungsgemäßen Vorrichtung mit einer Schwinggabel als mechanisch schwingfähige Einheit und einer zumindest zwei Leiterbahnen umfassenden elektromagnetisch schwingfähigen Einheit, wobei in (a) je eine Leiterbahn innerhalb eines Gabelzinkens, in (b) zwei Leiterbahnen im gleichen Gabelzinken und in (c) die zumindest zwei Leiterbahnen innerhalb der elektromagnetisch schwingfähigen Einheit angeordnet sind.

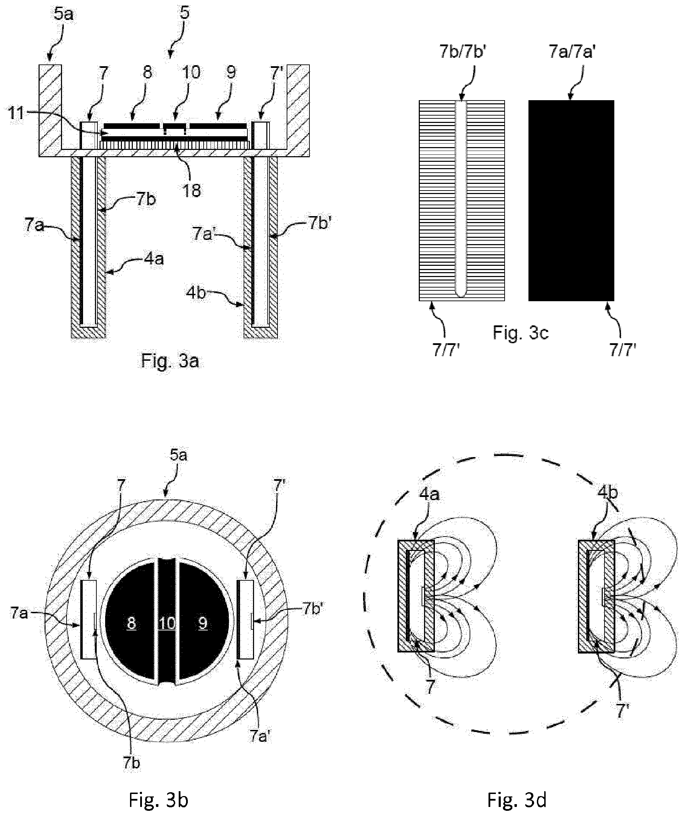

Fig. 3 eine Ausgestaltung einer erfindungsgemäßen Vorrichtung mit zumindest zwei elektromagnetisch schwingfähigen Einheiten, welche innerhalb einer mechanisch schwingfähigen Einheit in Form einer Schwinggabel angeordnet sind in (a) einer ersten Seitenansicht, (b) einer zweiten Seitenansicht, (c) eine Detailansicht der zumindest zwei Leiterbahnen einer der elektromagnetisch schwingfähigen Einheiten, und (d) eine mögliche Feldverteilung der sich von beiden elektromagnetisch schwingfähigen Einheiten ausgehend ausbreitenden elektromagnetischen Wellen,

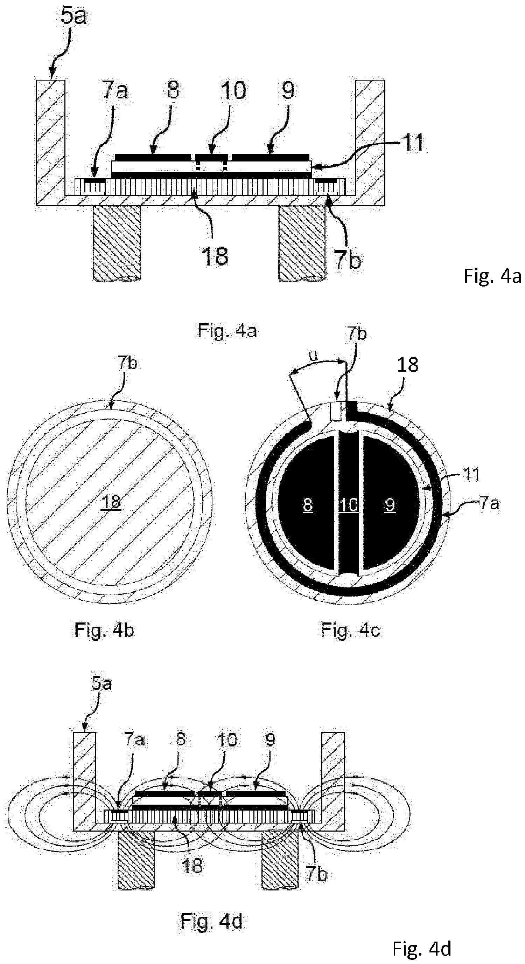

Fig. 4 eine Ausgestaltung einer erfindungsgemäßen Vorrichtung gemäß Fig. 2c) in (a) einer Seitenansicht, (b) eine Detailansicht der Antriebs-/Empfangseinheit sowie der zwei Leiterbahnen der elektromagnetisch schwingfähigen Einheit und (c) eine mögliche Feldverteilung der sich von der elektromagnetisch schwingfähigen Einheiten ausgehend ausbreitenden elektromagnetischen Wellen, und

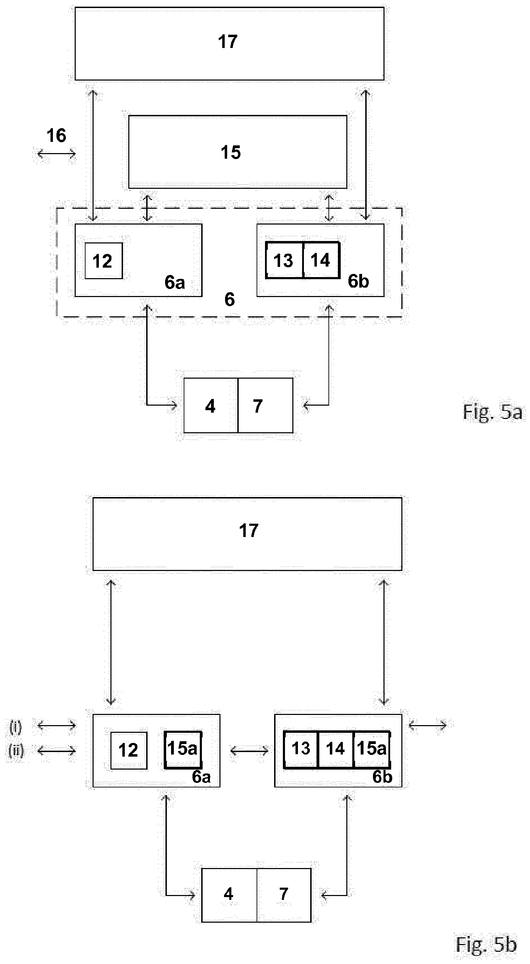

Fig. 5 (a-b) zwei mögliche Varianten für die elektronischen Komponenten einer erfindungsgemäßen Vorrichtung.

In Fig. 1 ist eine Vorrichtung 1 zur Bestimmung und/oder Überwachung zumindest einer Prozessgröße in Form eines vibronischen Sensors 1 gezeigt. Dargestellt ist eine mechanisch schwingfähige Einheit 4 in Form einer Schwinggabel, welche teilweise in ein Medium 2 eintaucht, welches sich in einem Behälter 3 befindet. Die nachfolgende Beschreibung wird sich ohne Beschränkung der Allgemeinheit weiterhin auf mechanisch schwingfähige Einheiten 4 in Form einer Schwinggabel beziehen. Insbesondere für die Ausgestaltungen, in welchen zumindest einer elektromagnetisch schwingfähige Einheit 7 zumindest teilweise innerhalb der mechanisch schwingfähigen Einheit 4 angeordnet ist, ist es von Vorteil, zumindest die zwei Gabelzinken der mechanisch schwingfähigen Einheit 4 aus einem Kunststoff zu fertigen.

Die schwingfähige Einheit 4 wird mittels der Antriebs-/Empfangseinheit 5 zu mechanischen Schwingungen angeregt, und kann beispielsweise ein piezoelektrischer Stapel- oder Bimorphantrieb sein. Es versteht sich jedoch von selbst, dass auch andere Ausgestaltungen eines vibronischen Sensors unter die Erfindung fallen. Weiterhin ist eine Elektronikeinheit 6 dargestellt, mittels welcher die Signalerfassung, -auswertung und/oder -speisung erfolgt.

Eine erfindungsgemäße Vorrichtung umfasst neben einer mechanisch schwingfähigen Einheit 4 ebenfalls eine elektromagnetisch schwingfähige Einheit 7. Bezüglich der Anordnung der mechanisch schwingfähigen Einheit 4 und der elektromagnetisch schwingfähigen Einheit 7 bestehen vielfältige Möglichkeiten. Eine besonders platzsparende Ausgestaltung ergibt sich, wenn die zumindest eine elektromagnetisch schwingfähige Einheit 7 zumindest teilweise an oder in zumindest einem Teilbereich einer mechanisch schwingfähigen Einheit 4 angeordnet wird. Auf diese Ausgestaltungsvariante wird sich die nachfolgende Beschreibung ohne Beschränkung der Allgemeinheit beziehen. Für den Fall einer mechanisch schwingfähigen Einheit 4 in Form einer Schwinggabel mit zwei Gabelzinken 4a, 4b und einer direkt an diese angrenzenden Antriebs-/Empfangseinheit 5 und einer elektromagnetisch schwingfähigen Einheit 7 mit zumindest zwei Leiterbahnen 7a, 7b sind beispielhaft drei verschiedene Ausführungsformen in Fig. 2 gezeigt. Die jeweiligen Überlegungen lassen sich leicht auch auf andere Ausgestaltungen der zumindest einen mechanisch schwingfähigen Einheit 4 und der zumindest einen elektromagnetisch schwingfähigen Einheit 7 übertragen.

In der Ausführung gemäß Fig. 2a ist die erste Leiterbahn 7a der elektromagnetisch schwingfähigen Einheit 7 im ersten Gabelzinken 4a der mechanisch schwingfähigen Einheit 4 angeordnet, während sich die zweite Leiterbahn 7b im zweiten Gabelzinken 4b befindet. In diesem Beispiel handelt es sich um eine symmetrische Anordnung zweier gleich ausgestalteter Leiterbahnen 7a, 7b, welche vollständig durch die beiden Gabelzinken 4a, 4b gegenüber dem jeweiligen Medium isoliert sind. Dagegen sind für die Ausführung gemäß Fig. 2b beide Leiterbahnen 7a, 7b der elektromagnetisch schwingfähigen Einheit 7 im ersten Gabelzinken 4a der mechanisch schwingfähigen Einheit 4 angeordnet und für das Ausführungsbeispiel gemäß Fig. 2c ist die elektromagnetisch magnetisch schwingfähige Einheit 7 schließlich vollständig innerhalb des Gehäuses 5a der Antriebs-/Empfangseinheit 5 positioniert.

Für alle in Fig. 2 gezeigten Beispiele ist jeweils eine elektromagnetisch schwingfähige Einheit 7 innerhalb einer mechanisch schwingfähigen Einheit 4 bzw. innerhalb des Gehäuses 5a der dazugehörigen Antriebs-/Empfangseinheit 5 angeordnet. Es kann jedoch auch mehr als eine elektromagnetisch schwingfähige Einheit 7 vorgesehen sein. Insbesondere können auch mehrere elektromagnetisch schwingfähige Einheiten 7 innerhalb derselben mechanisch schwingfähigen Einheit 4 angeordnet sein, wie für den in Fig. 3 gezeigten Fall von zwei elektromagnetisch schwingfähigen Einheiten 7, 7', welche in den beiden Gabelzinken 4a,4b einer mechanisch schwingfähigen Einheit 4 in Form einer Schwinggabel angeordnet sind. Eine Seitenansicht eines Beispiels für eine derartige Vorrichtung ist in Fig. 3a gezeigt. Eine erste elektromagnetisch schwingfähige Einheit 7 mit zwei Leiterbahnen 7a, 7b ist innerhalb des ersten Gabelzinkens 4a der mechanisch schwingfähigen Einheit 4 angeordnet, ähnlich wie im in Fig. 2b) gezeigten Beispiel. Allerdings ist gemäß Fig. 3a eine zweite elektromagnetisch schwingfähige Einheit 7', ebenfalls mit zwei Leiterbahnen 7a' und 7b', vorgesehen, welche sich innerhalb des zweiten Gabelzinkens 4b befindet. Die beiden elektromagnetisch schwingfähigen Einheiten 7, 7' sind gleichartig ausgestaltet und symmetrisch zueinander innerhalb der beiden Gabelzinken 4a, 4b positioniert, um das Schwingungsverhalten der mechanisch schwingfähigen Einheit 4 nicht zu beeinflussen. Eine symmetrische Anordnung der zumindest einen elektromagnetisch schwingfähigen Einheit 7 innerhalb der mechanisch schwingfähigen Einheit 4 ist deshalb generell bevorzugt.

Die Antriebs-/Empfangseinheit 5 mit dem Gehäuse 5a umfasst für das in Fig. 3 gezeigte Beispiel einen sogenannten piezoelektrischen Bimorph-Antrieb mit einem scheibenförmigen piezoelektrischen Element 11, auf dessen einer Seitenfläche in einem ersten Bereich eine Sendeelektrode 8 und in einem zweiten Bereich eine Empfangselektrode 9 angeordnet sind. Die Sendeelektrode 8 und Empfangselektrode 9 sind hier formgleich ausgestaltet und achsensymmetrisch zueinander ausgerichtet. Ferner befindet sich in einem dritten Bereich symmetrisch um die Spiegelachse der Sendeelektrode 8 und Empfangselektrode 9 verlaufenden Bereich eine Referenzelektrode 10, welche entweder auf undefiniertem Potential liegen, oder beispielsweise als Massepotential dienen kann. Unterhalb des piezoelektrischen Elements 11 ist ferner eine Steatitscheibe 18 handeln. Hierbei kann auch ein anderes Material, insbesondere ein Leiterplattenmaterial verwendet werden. Es versteht sich ferner von selbst, dass auch andere Ausgestaltungen der Antriebs-/Empfangseinheit 5 möglich sind.

In Fig. 3b ist eine gegenüber der Ansicht aus Fig.3a um 90° gedrehte zweidimensionale Ansicht der gleichen Anordnung gezeigt. Aus dieser Perspektive lässt sich erkennen, dass für jede der zwei elektromagnetisch schwingfähigen Einheiten 7, 7' jeweils die Geometrie die erste Leiterbahn 7a, 7a' und die zweite Leiterbahn 7b, 7b' unterscheiden. Diese Unterschiede sind in der in Fig. 3c gezeigten Detailansicht der beiden Leiterbahnen 7a, 7a', 7b, 7b' der elektromagnetisch schwingfähigen Einheit 7, 7' noch deutlicher ersichtlich. Die erste Leiterbahn 7a, 7a' ist jeweils schmaler in Bezug auf die Breite eines Gabelzinkens 4a, 4b der mechanisch schwingfähigen Einheit 4 als die zweite Leiterbahn 7b, 7b'. Jede der beiden elektromagnetisch schwingfähigen Einheiten 7, 7' lässt sich somit aus zwei Leiterbahnlagen zusammenfassen, welche beispielsweise durch eine Kunststoffschicht voneinander isoliert sind. Beispielsweise kann es sich dabei um eine zweilagige Leiterplatte handeln. Jeweils eine der beiden Leiterbahnen 7a, 7a' oder 7b, 7b' ist mit der Erde oder Masse verbunden ist. In dem Beispiel gemäß Fig. 3 ist dies jeweils die breiter ausgeführte zweite Leiterbahn 7b, 7b'. Die elektromagnetisch schwingfähigen Einheiten 7, 7' sind vorteilhaft von einem Kunststoff umgeben, wodurch neben einer Schutzwirkung auch eine galvanische Trennung zum jeweiligen Medium 2 sichergestellt werden kann. Eine solche galvanische Trennung muss dann nicht mehr innerhalb der elektronischen Komponenten 6 der Vorrichtung 1 ausgeführt werden.

Durch die in den Fig. 3a-3c gezeigte Ausführung einer erfindungsgemäßen Vorrichtung ergibt sich für das elektromagnetische Feld eine Feldverteilung, wie in Fig. 3d schematisch gezeigt ist. Für jede der beiden elektromagnetisch schwingfähigen Einheiten 7, 7' ergibt sich im Raumbereich ausgehend von der jeweiligen zweiten Leiterbahn 7b, 7b' eine vergleichsweise hohe Felddichte. Gemäß Fig. 3d ist die erste elektromagnetisch schwingfähige Einheit 7 beispielsweise sensitiv für den Zwischenraum zwischen den beiden Gabelzinken 4a, 4b, während die zweite elektromagnetisch schwingfähige Einheit 7' sensitiv für einen Raumbereich außerhalb des Zwischenraums zwischen den beiden Gabelzinken 4a, 4b ist. Dann lässt sich beispielsweise vorteilhaft mittels der zweiten elektromagnetisch schwingfähigen Einheit 7' die Permittivität, Permeabilität, der Brechungsindex oder auch der dielektrische Verlustfaktor bestimmen. Aus einer Differenz eines Vergleichswerts für die jeweilige Prozessgröße, welche anhand der ersten elektromagnetisch schwingfähigen Einheit 7 ermittelt wird, kann ferner auf die Bildung von Ansatz zwischen den beiden Gabelzinken 4a, 4b geschlossen werden. Es sei darauf verwiesen, dass die Geometrie der elektromagnetisch schwingfähigen Einheiten 7, 7' durch die jeweilige geometrische Ausgestaltung anwendungsbezogen optimiert werden kann, beispielsweise derart, dass ein möglichst großer Raumbereich oder ein kleinerer Raumbereich möglichst exakt erfassbar, sprich durch das jeweilige elektromagnetische Feld durchsetzt, wird. Es versteht sich von selbst, dass anstelle der parallelen Ausrichtungen der beiden elektromagnetisch schwingfähigen Einheiten 7, 7', bei welcher jeweils die erste breit ausgeführte Leiterbahn 7a, 7a' links und die zweite schmal ausgeführte Leiterbahn 7b, 7b' rechts angeordnet sind, beispielsweise auch eine antiparallele Anordnung der beiden elektromagnetisch schwingfähigen Einheiten 7, 7' möglich ist, so dass beide elektromagnetisch schwingfähigen Einheiten für einen Raumbereich außerhalb des Zwischenraums der beiden Gabelzinken 4a, 4b sensitiv sind. In diesem Falle lässt sich beispielsweise neben der eigentlich Prozessgrößenbestimmung differentieller Ansatz an den Außenseiten der Gabelzinken 4a, 4b feststellen. Die Kenntnis über Ansatzbildung kann sich dabei vorteilhaft auf die Messgenauigkeit des Messgeräts auswirken.

Eine weitere mögliche Ausgestaltung einer vorliegenden Erfindung ist schließlich Gegenstand von Fig. 4. Die Antriebs-/Empfangseinheit 5 umfasst, wie in der Ausgestaltung gemäß Fig. 3 einen piezoelektrischen Bimorphantrieb. Wie aus Fig. 4a ersichtlich, ist eine elektromagnetisch schwingfähige Einheit 7 innerhalb des Gehäuses 5a der Antriebs-/Empfangseinheit 5 in Form von zwei im Wesentlichen ringförmigen Leiterbahnen 7a, 7b, welche sich auf zwei sich gegenüberliegenden Seitenflächen der Steatitscheibe 18 befinden, angeordnet. Die exakte Anordnung der elektromagnetisch schwingfähigen Einheit 7 lässt sich jedoch genauer anhand der Detailzeichnungen in Fig. 4b und Fig.4c erkennen. Gemäß des in Fig. 4b gezeigten Beispiels ist die zweite Leiterbahn7b ringförmig ausgestaltet und im Bereich einer ersten, in diesem Beispiel kreisrunden, Seitenfläche der Steatitscheibe 18 angeordnet. Anstelle der Steatischeibe 18 kann beispielsweise auch eine Leiterplatte verwendet werden. Die zweite Leiterbahn 7b ist ferner über eine elektrisch leitfähige Kontaktierung von der ersten zur dieser gegenüberliegenden zweiten Seitenfläche der Steatitscheibe 18 hin kontaktiert. Auf dieser zweiten Seitenfläche ist außerdem die erste Leiterbahn 7a in Form eines nicht geschlossenen Ringabschnitts mit einer Ausnehmung der Länge u angeordnet, welche erste Leiterbahn teilweise um das piezoelektrische Element 11 mit den Elektroden 8,9 und 10 herum verläuft. Die Länge der Ausnehmung u kann dabei je nach Anwendungsfall geeignet gewählt werden. Es sei darauf verwiesen, dass für das hier gezeigte Ausführungsbeispiel grundsätzlich viele verschiedene Abwandlungsmöglichkeiten bestehen. Beispielsweise kann die zweite Leiterbahn auch in Form eines Vollkreises oder in Form eines Kreisausschnitts, aber auch in Form einer zweidimensionalen geometrischen Fläche andere Geometrie ausgeführt sein. Auch können die Leiterbahnen 7a,7b andersartig innerhalb des Gehäuses der Antriebs-/Empfangseinheit angeordnet sein, bzw. es können auch andersartig ausgestaltete elektromagnetisch schwingfähige Einheiten 7 eingesetzt werden.

Der geringe Abstand der beiden Leiterbahnen 7a,7b für dieses Beispiel erlaubt es, eine vergleichsweise hohe Frequenz zu wählen, wodurch sich das jeweilige elektromagnetische Feld nur in einem vergleichsweise kleinen Raumbereich ausbreitet und woraus eine hohe Empfindlichkeit der Anordnung resultiert. Die sich ergebende Feldverteilung ist schließlich in Fig. 4c skizziert. Je nach geometrischer Auslegung der elektromagnetisch schwingfähigen Einheit 7 kann eine derartige Anordnung auch zur Erkennung des Eindringens von Medium in das Gehäuse 5a der Antriebs-/Empfangseinheit verwendet werden.

Schließlich sind in den Figuren Fig. 5a und Fig. 5b zwei mögliche Ausgestaltungen für die elektronischen Komponenten einer erfindungsgemäßen Vorrichtung in Form zweier Blockschaltbilder gezeigt. Im Beispiel gemäß Fig. 5a sind eine erste 6a und eine zweite Elektronikeinheit 6b vorgesehen. Die erste Elektronikeinheit 6a ist mit der mechanisch schwingfähigen Einheit 4 mittels der Antriebs-/Empfangseinheit (hier nicht extra dargestellt) verbunden und umfasst zumindest die Regeleinheit 12, welche zumindest dazu ausgestaltet ist, das Anregesignal ausgehend vom Empfangssignal zu erzeugen, und eine vorgebbare Phasenverschiebung zwischen dem Anregesignal und dem Empfangssignal einzustellen. Die zweite Elektronikeinheit 6b ist wiederum mit der elektromagnetisch schwingfähigen Einheit 7 verbunden und umfasst zumindest das aktive Element 13 und die Auskoppeleinheit 14. Die beiden Elektronikeinheiten 6a, 6b sind wiederum beide mit einer Auswerteeinheit 15 verbunden, innerhalb welcher zumindest die zumindest eine Prozessgröße bestimmt wird. Dabei umfasst die Auswerteeinheit 15 beispielsweise einen Mikroprozessor. Außerdem kann sie noch eine Speichereinheit aufweisen. Die Auswerteeinheit 15 kann ferner mit einem übergeordneten Leitsystem verbunden sein 16.

Optional kann darüber hinaus noch eine Triggereinheit 17 integriert werden. Dies eignet sich besonders im Falle eines gepulsten Betriebs der elektromagnetisch schwingfähigen Einheit 7. Diese Einheit triggert zumindest die Auskopplung des Ausgangssignals auf ein bestimmtes anhand des Anregesignals als Funktion der Zeit ermittelbares Zeitintervall, insbesondere auf ein einen Nulldurchgang oder einen Extrem-Wert des Anregesignal umfassendes Zeitintervall. Ein derartig getriggerter Betrieb zeichnet sich zum einen durch eine besonders geringe Leistungsaufnahme aus. Zum anderen wird eine mögliche gegenseitige Beeinflussung des Ausgangssignals und des Anrege- und/oder Empfangssignals minimiert.

Alternativ zu dem in Fig. 5a gezeigten Beispiel können die erste und zweite Elektronikeinheit auch in einer übergeordneten Elektronikeinheit 6 (gestricheltes Kästchen) zusammengefügt werden. Auch die Auswerteeinheit 15 sowie die Triggereinheit 17 können in dieser Elektronikeinheit 6 angeordnet sein.

Eine zweite Variante für eine erfindungsgemäße Anordnung der elektronischen Komponenten ist in Fig. 5b dargestellt. In diesem Falle sind zwei getrennte Auswerteeinheiten 15a, 15b vorgesehen, wobei die erste Auswerteeinheit 15a der ersten Elektronikeinheit 6a zugeordnet ist, und zur Bestimmung einer Prozessgröße zumindest aus dem Empfangssignal der mechanisch schwingfähigen Einheit 4 ausgestaltet ist, und wobei die zweite Auswerteeinheit 15b der zweiten Elektronikeinheit 6b zugeordnet ist, und zur Bestimmung einer Prozessgröße zumindest aus dem Ausgangssignal der elektromagnetisch schwingfähigen Einheit 7 ausgelegt ist. Die beiden Auswerteeinheiten 15a, 15b können selbstverständlich auch separat von den beiden Elektronikeinheiten 6a, 6b angeordnet werden. Wieder kann optional auch eine Triggereinheit 17 integriert werden.

Für eine gegebenenfalls gewünschte Verbindung mit einem übergeordneten Leitsystem sind bei dieser Ausgestaltung mehrere Varianten denkbar. Entweder jede der zwei Auswerteeinheiten 15a, 15b ist mit dem übergeordneten Leitsystem verbunden (Fall i). Alternativ können die beiden Auswerteeinheiten 15a, 15b jedoch auch untereinander verbunden sein (Fall ii). Dann reicht es aus, wenn eine der beiden Auswerteeinheiten 15a,15b, im hier gezeigten Beispiel die erste Auswerteeinheit 15a, mit einem übergeordneten Leitsystem verbunden ist. Die Kommunikation zwischen den beiden Auswerteeinheiten 15a,15b kann dann beispielsweise nach dem Master/Slave-Prinzip erfolgen.

**Bezugszeichenliste**

| | |
|---|---|
| 1 | Vibronischer Sensor |
| 2 | Medium |
| 3 | Behälter |
| 4 | Mechanisch schwingfähige Einheit, Schwinggabel |
| 4a | erster Gabelzinken |
| 4b | zweiter Gabelzinken |
| 5 | Antriebs-/Empfangseinheit |
| 5a | Gehäuse der Antriebs-/Empfangseinheit |
| 6 | Elektronikeinheit |
| 6a | erste Elektronikeinheit |
| 6b | zweite Elektronikeinheit |
| 7 | elektromagnetisch schwingfähige Einheit |
| 7a | erste Leiterbahn |
| 7b | zweite Leiterbahn |
| 8 | Sendeelektrode |
| 9 | Empfangselektrode |
| 10 | Referenzelektrode |
| 11 | Piezoelektrisches Element |
| 12 | Regeleinheit |
| 13 | aktives Element |
| 14 | Auskoppeleinheit |
| 15 | Auswerteeinheit |
| 15a | erste Auswerteeinheit |
| 15 | bzweite Auswerteeinheit |
| 16 | Verbindung zu übergeordneten Leitsystem |
| 17 | Triggereinheit |
| 18 | Steatitscheibe |
| u | Länge der Ausnehmung einer in Form eines Ringabschnitts vorliegenden Leiterbahn |

**Patentansprüche**

1. Vorrichtung zur Bestimmung und/oder Überwachung zumindest einer Prozessgröße eines Mediums (2) in einem Behälter (3) mit zumindest

- einer mechanisch schwingfähigen Einheit (4), und
- einer Antriebs-/Empfangseinheit (5) zur Anregung der mechanisch schwingfähigen Einheit (4) zu mechanischen Schwingungen mittels eines elektrischen Anregesignals und zum Empfangen und Umwandeln der mechanischen Schwingungen in ein elektrisches Empfangssignal
- einer Regeleinheit (12), welche dazu ausgestaltet ist, das Anregesignal ausgehend vom Empfangssignal zu erzeugen, und eine vorgebbare Phasenverschiebung zwischen dem Anregesignal und dem Empfangssignal einzustellen,
- einer elektromagnetisch schwingfähigen Einheit (7),
- einem aktiven Element (13) zur Erzeugung und/oder Aufrechterhaltung der elektromagnetischen Schwingungen in der elektromagnetisch schwingfähigen Einheit (7), welches aktive Element (13) zusammen mit der elektromagnetisch schwingfähigen Einheit (7) einen Oszillator bildet
- einer Auskoppeleinheit (14), welche dazu ausgestaltet ist, über das aktive Element (13) ein Ausgangssignal abzugreifen, und
- einer Auswerteeinheit (15),

welche Auswerteeinheit (15) dazu ausgestaltet ist, die zumindest eine Prozessgröße aus dem Empfangssignal und/oder aus dem Ausgangssignal zu bestimmen,
wobei zumindest eine mechanisch schwingfähige Einheit (4) in Form einer Schwinggabel mit einem ersten Gabelzinken (4a) und einem zweiten Gabelzinken (4b) und zumindest eine elektromagnetisch schwingfähige Einheit (7) mit zumindest einer ersten Leiterbahn (7a) und einer zweiten Leiterbahn (7a) vorgesehen sind, und
wobei die erste Leiterbahn (7a) zumindest einer der zumindest einen elektromagnetisch schwingfähigen Einheit (7) zumindest teilweise im Innenraum des ersten Gabelzinkens (4a) und die zweite Leiterbahn (7b) dieser elektromagnetisch schwingfähigen Einheit (7) zumindest teilweise im Innenraum des zweiten Gabelzinkens (4a) der zumindest einen mechanischen schwingfähigen Einheit (4) angeordnet ist oder
wobei die erste (7a) und zweite Leiterbahn (7b) zumindest einer der zumindest einen elektromagnetisch schwingfähigen Einheit (7) zumindest teilweise im Innenraum desselben Gabelzinkens (4a,4b) einer der zumindest einen mechanischen schwingfähigen Einheit (4) angeordnet sind, oder
wobei die erste (7a) und/oder zweite Leiterbahn (7b) zumindest einer elektromagnetisch schwingfähigen Einheit (7) innerhalb eines Gehäuses (5a) der Antriebs-/Empfangseinheit (5) angeordnet sind.

2. Vorrichtung nach Anspruch 1,
wobei die mechanisch schwingfähige Einheit (4) aus einem Metall, einer Keramik oder aus einem Kunststoff gefertigt ist.

3. Vorrichtung nach zumindest einem der vorhergehenden Ansprüche, wobei die Antriebs-/Empfangseinheit (5) zumindest ein piezoelektrisches Element (11) oder zumindest eine Spule umfasst.

4. Vorrichtung nach zumindest einem der vorhergehenden Ansprüche,
wobei die elektromagnetisch schwingfähige Einheit (7) als Wellenleiter, insb. als Koaxialkabel, Hohlraumleiter, oder Mikrostreifenleitung, als Patchantenne, als dielektrischer Gradientenleiter oder als Zweidrahtleitung ausgebildet ist

5. Vorrichtung nach zumindest einem der vorhergehenden Ansprüche,
wobei die zumindest eine Prozessgröße gegeben ist durch einen Füllstand oder Grenzstand des Mediums in dem Behälter, durch die Viskosität, die Dichte, die Permittivität, die Permeabilität, den dielektrischen Verlustfaktor, den Eiweißgehalt und/oder Fettgehalt eines Mediums, insb. eines Lebensmittels, oder durch den Wasseranteil in einem Öl.

6. Vorrichtung nach zumindest einem der vorhergehenden Ansprüche,
wobei zumindest zwei elektromagnetisch schwingfähige Einheiten (7,7') vorgesehen sind, und wobei die zumindest zwei elektromagnetisch schwingfähigen Einheiten (7,7') derart angeordnet sind und die zumindest eine Auswerteeinheit (15) dazu ausgestaltet ist, aus einem Vergleich zumindest eines ersten Ausgangssignals der ersten elektromagnetisch schwingfähigen Einheit (7) und einem zweiten Ausgangssignal der zweiten elektromagnetisch schwingfähigen Einheit (7') auf einen Ansatz an der zumindest einen mechanisch schwingfähigen Einheit (4) zu schließen.

7. Vorrichtung nach zumindest einem der vorhergehenden Ansprüche,
wobei zumindest eine elektromagnetisch schwingfähige Einheit (7) in einem Gehäuse (5a) der Antriebs-/Empfangseinheit (5) angeordnet ist, und wobei die Auswerteeinheit (15) dazu ausgestaltet ist, aus dem Ausgangssignal der elektromagnetisch schwingfähigen Einheit (7) auf das Eindringen von Medium in das Gehäuse (5a) der Antriebs-/Empfangseinheit (5) zu schließen.

8. Vorrichtung nach zumindest einem der vorhergehenden Ansprüche,
mit einem Temperatursensor zur Erfassung der Temperatur des Mediums.

9. Vorrichtung nach zumindest einem der vorhergehenden Ansprüche,
wobei die elektromagnetisch schwingfähige Einheit (7) mit einer Frequenz im Bereich von 100MHz bis 100GHz schwingt und/oder wobei die mechanisch schwingfähige Einheit (4) mit einer Frequenz im Bereich von 100Hz bis 100kHz schwingt.

10. Vorrichtung nach zumindest einem der vorhergehenden Ansprüche,

wobei eine Triggereinheit (17) vorgesehen ist, welche Triggereinheit (17) dazu ausgestaltet ist, zumindest die Auskopplung des Ausgangssignals auf ein bestimmtes anhand des Anregesignals als Funktion der Zeit ermittelbares Zeitintervall, insbesondere ein einen Nulldurchgang oder einen Extrem-Wert des Anregesignal umfassendes Zeitintervall, zu triggern.

**Claims**

1.  Apparatus designed to determine and/or monitor at least a process variable of a medium (2) in a vessel (3) with at least

    - a unit capable of mechanical vibration (4), and
    - a drive/reception unit (5) designed to excite the unit capable of mechanical vibration (4) to produce mechanical vibrations using an electrical excitation signal and designed to receive and convert the mechanical vibrations into an electrical reception signal
    - a control unit (12), which is designed to generate the excitation signal on the basis of the reception signal, and to set a predefinable phase shift between the excitation signal and the reception signal,
    - a unit capable of electromagnetic vibration (7),
    - an active element (13) designed to generate and/or maintain electromagnetic vibrations in the unit capable of electromagnetic vibration (7), wherein said active element (7) forms an oscillator together with the unit capable of electromagnetic vibration (7),
    - a decoupling unit (14), which is designed to pick up an output signal via the active element (13), and
    - an evaluation unit (15),

    wherein said evaluation unit (15) is designed to determine the at least one process variable from the reception signal and/or from the output signal, wherein at least a unit capable of mechanical vibration (4) is provided in the form of a tuning fork with a first fork tine (4a) and a second fork tine (4b) and at least a unit capable of electromagnetic vibration (7) with at least a first conductive track (7a) and a second conductive track (7b), and
    wherein the first conductive track (7a) of at least one of the at least one unit capable of electromagnetic vibration (7) is arranged at least partially in the interior of the first fork tine (4a) and the second conductive track (7b) of said unit capable of electromagnetic vibration (7) is arranged at least partially in the interior of the second fork tine (4b) of the at least one unit capable of mechanical vibration (4),
    or
    wherein the first (7a) and the second conductive track (7b) of at least one of the at least one unit capable of electromagnetic vibration (7) is arranged at least partially in the interior of the same fork tine (4a ,4b) of one of the at least one unit capable of mechanical vibration (4), or
    wherein the first (7a) and/or the second conductive track (7b) of at least a unit capable of electromagnetic vibration (7) is arranged inside a housing (5a) of the drive/reception unit (5).

2.  Apparatus as claimed in Claim 1,
    wherein the unit capable of mechanical vibration (4) is made from a metal, a ceramic or a plastic material.

3.  Apparatus as claimed in at least one of the previous claims,
    wherein the drive/reception unit (5) comprises at least a piezoelectric element (11) or at least a coil.

4.  Apparatus as claimed in at least one of the previous claims,
    wherein the unit capable of electromagnetic vibration (7) is designed as a waveguide, particularly a coaxial cable, a hollow waveguide or a microstrip, as a patch antenna, as a dielectric gradient conductor or a two-wire cable.

5.  Apparatus as claimed in at least one of the previous claims,
    wherein the at least one process variable is defined by a level or point level of the medium in the vessel, by the viscosity, the density, the permittivity, the permeability, the dielectric loss factor, the protein content and/or the fat content of a medium, particularly a food, or by the water content in an oil.

6.  Apparatus as claimed in at least one of the previous claims,
    wherein at least two units capable of electromagnetic vibration (7, 7') are provided, and wherein the at least two units capable of electromagnetic vibration (7, 7') are arranged in such a way - and the at least one evaluation unit (15) is designed in such a way - to deduce, on the basis of a comparison of at least a first output signal of the first unit capable of electromagnetic vibration (7) and of a second output signal of the second unit capable of electromagnetic vibration (7'), the presence of deposit buildup on the at least one unit capable of mechanical vibration (4).

7. Apparatus as claimed in at least one of the previous claims,
wherein at least one unit capable of electromagnetic vibration (7) is arranged in a housing of the drive/reception unit (5), and the evaluation unit (15) is designed to deduce, on the basis of the output signal of the unit capable of electromagnetic vibration (7), the penetration of medium into the housing (5a) of the drive/reception unit (5).

8. Apparatus as claimed in at least one of the previous claims,
with a temperature sensor designed to measure the temperature of the medium.

9. Apparatus as claimed in at least one of the previous claims,
wherein the unit capable of electromagnetic vibration (7) vibrates at a frequency in the range from 100 MHz to 100 GHz and/or wherein the unit capable of mechanical vibration (4) vibrates at a frequency in the range from 100 Hz to 100 kHz.

10. Apparatus as claimed in at least one of the previous claims,
wherein a trigger unit (17) is provided, said trigger unit (17) being designed to trigger at least the decoupling of the output signal at a specific time interval, which can be determined on the basis of the excitation signal as a function of time, particularly a time interval comprising a zero crossing or an extreme value of the excitation signal.

**Revendications**

1. Dispositif destiné à la détermination et/ou la surveillance d'au moins une grandeur process d'un produit (2) dans un réservoir (3) avec au moins

   - une unité apte à vibrer mécaniquement (4), et
   - une unité d'entraînement / de réception (5) destinée à exciter l'unité apte à vibrer mécaniquement (4) en vibrations mécaniques au moyen d'un signal d'excitation électrique et destinée à la réception et à la conversion des vibrations mécaniques en un signal de réception électrique
   - une unité de réglage (12), laquelle est conçue de telle sorte à générer le signal d'excitation à partir du signal de réception, et à régler un décalage de phase prédéfinissable entre le signal d'excitation et le signal de réception,
   - une unité apte à vibrer électromagnétiquement (7),
   - un élément actif (13) destiné à la génération et/ou au maintien des vibrations électromagnétiques dans l'unité apte à vibrer électromagnétiquement (7), lequel élément actif (7) forme un oscillateur,
   - une unité de découplage (14), laquelle est conçue de telle sorte à prélever un signal de sortie à travers l'élément actif (13), et
   - une unité d'exploitation (15),

   laquelle unité d'exploitation (15) est conçue de telle sorte à déterminer l'au moins une grandeur de process à partir du signal de réception et/ou à partir du signal de sortie,
   au moins une unité apte à vibrer mécaniquement (4) étant prévue sous la forme d'une fourche vibrante avec un premier bras de fourche (4a) et un deuxième bras de fourche (4b) et au moins une unité apte à vibrer électromagnétiquement (7) avec au moins une première piste conductrice (7a) et une deuxième piste conductrice (7b), et
   la première piste conductrice (7a) d'au moins l'une des au moins une unité apte à vibrer électromagnétiquement (7) étant disposée au moins partiellement à l'intérieur du premier bras de fourche (4a) et la deuxième piste conductrice (7b) de cette unité apte à vibrer électromagnétiquement (7) étant disposée au moins partiellement à l'intérieur du deuxième bras de fourche (4b) de l'au moins une unité apte à vibrer mécaniquement (4), ou
   la première (7a) et la deuxième piste conductrice (7b) d'au moins l'une des au moins une unité apte à vibrer électromagnétiquement (7) étant disposées au moins partiellement à l'intérieur du même bras de fourche (4a ,4b) de l'une des au moins une unité apte à vibrer mécaniquement (4), ou
   la première (7a) et/ou la deuxième piste conductrice (7b) d'au moins une unité apte à vibrer électromagnétiquement (7) étant disposée à l'intérieur d'un boîtier (5a) de l'unité d'entraînement / de réception (5).

2. Dispositif selon la revendication 1,
pour lequel l'unité apte à vibrer mécaniquement (4) est fabriquée en un métal, une céramique ou une matière plastique.

**3.** Dispositif selon au moins l'une des revendications précédentes,
pour lequel l'unité d'entraînement / de réception (5) comprend au moins un élément piézoélectrique (11) ou au moins une bobine.

**4.** Dispositif selon au moins l'une des revendications précédentes,
pour lequel l'unité apte à vibrer électromagnétiquement (7) est conçue en tant que guide d'ondes, notamment un câble coaxial, un guide d'ondes creux ou un guide d'ondes à ruban, en tant qu'antenne patch, en tant que guide à gradient d'indice diélectrique ou en tant que câble bifilaire.

**5.** Dispositif selon au moins l'une des revendications précédentes,
pour lequel l'au moins une grandeur de process est définie par un niveau de remplissage ou un seuil de niveau du produit dans le réservoir, par la viscosité, la densité, la permittivité, la perméabilité, le facteur de perte diélectrique, la teneur en protéines et/ou la teneur en graisses d'un produit, notamment un produit alimentaire, ou par la teneur en eau d'une huile.

**6.** Dispositif selon au moins l'une des revendications précédentes,
pour lequel sont prévues au moins deux unités aptes à vibrer électromagnétiquement (7, 7'), et pour lequel les au moins deux unités aptes à vibrer électromagnétiquement (7, 7') sont disposées de telle manière - et l'au moins une unité d'exploitation (15) est conçue de telle sorte - à déduire, à partir d'une comparaison d'au moins un premier signal de sortie de la première unité apte à vibrer électromagnétiquement (7) et d'un deuxième signal de sortie de la deuxième unité apte à vibrer électromagnétiquement (7'), l'existence d'un dépôt sur l'au moins une unité apte à vibrer mécaniquement (4).

**7.** Dispositif selon au moins l'une des revendications précédentes,
pour lequel au moins une unité apte à vibrer électromagnétiquement (7) est disposée dans un boîtier de l'unité d'entraînement / de réception (5), et l'unité d'exploitation (15) étant conçue de telle sorte à déduire du signal de sortie de l'unité apte à vibrer électromagnétiquement (7) la pénétration de produit dans le boîtier (5a) de l'unité d'entraînement / de réception (5).

**8.** Dispositif selon au moins l'une des revendications précédentes,
avec un capteur de température destiné à la mesure de la température du produit.

**9.** Dispositif selon au moins l'une des revendications précédentes,
pour lequel l'unité apte à vibrer électromagnétiquement (7) vibre à une fréquence dans la gamme de 100 MHz à 100 GHz et/ou pour lequel l'unité apte à vibrer mécaniquement (4) vibre à une fréquence dans la gamme de 100 Hz à 100 kHz.

**10.** Dispositif selon au moins l'une des revendications précédentes,
pour lequel une unité de déclenchement (17) est prévue, laquelle unité de déclenchement (17) est conçue de telle sorte à déclencher au moins le découplage du signal de sortie à un intervalle de temps donné et pouvant être déterminé sur la base du signal d'excitation en fonction du temps, notamment un intervalle de temps incluant un passage par zéro ou une valeur extrême du signal d'excitation.

Fig. 1

Fig. 2a    Fig. 2b    Fig. 2c

Fig. 3a

Fig. 3c

Fig. 3b

Fig. 3d

Fig. 4a

Fig. 4a

Fig. 4b

Fig. 4c

Fig. 4d

Fig. 4d

Fig. 5a

Fig. 5b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006034105 A1
- DE 102007013557 A1
- DE 102005015547 A1
- DE 102009026685 A1
- DE 102009028022 A1
- DE 102010030982 A1
- DE 00102010030982 A1
- DE 10057974 A1
- DE 102006033819 A1
- DE 10050299 A1
- DE 102007043811 A1
- WO 2008006391 A1
- DE 102004059050 A1
- DE 102005050400 A1

- US 20060031030 A1
- DE 102012104075 A1
- DE 102013112025 A1
- DE 102013112026 A1
- DE 102011078060 A1
- EP 0751380 B1
- EP 0875741 B1
- EP 1134038 A1
- EP 1277243 B1
- EP 19969005 B1
- EP 1281051 B1
- WO 2007113011 A
- WO 2007114950 A1